# EUROPEAN PATENT APPLICATION

(11) **EP 4 691 342 A1**
(43) Date of publication of application: **11.02.2026**
(21) Application number: 24784561.3
(22) Date of filing: 06.01.2024
(51) Int. Cl.: A61B 3/06, A61B 10/00, G02C 11/00

(54) **CHARACTERISTIC INFORMATION COLLECTION METHOD**

(30) Priority: 01.04.2023 JP 2023059673
(71) Applicant: Kunkasha Co.,Ltd., Osaka-shi, Osaka, 530-0047 (JP)
(72) Inventor: MUKAI, Tadashi, Osaka-shi Osaka 530-0047 (JP)
(74) Representative: Weickmann & Weickmann PartmbB
(86) International application number: PCT/JP2024/000089
(87) International publication number: WO 2024/209757

(57) **Abstract**

A sensory traits information collecting method incudes: a first tasting step of including, in a mouth of a subject, predetermined food and drink (F) exhibiting predetermined oral sensation in a white light environment in which white light (LO) is incident on a retina (R) of the subject; a predetermined light environment creating step of creating a predetermined light environment in which predetermined light (L1) is incident on the retina (R) of the subject; a second tasting step of again including the predetermined food and drink (F) in the mouth of the subject in the predetermined light environment; an information reception step of receiving sensory traits information regarding oral sensation of the predetermined food and drink (F) in the first tasting step and the second tasting step from the subject; and a collecting step of collecting the sensory traits information received from the subject.

## Description

### [Technical Field]

The present invention relates to a sensory traits information collecting method for collecting sensory traits information regarding integration of vision and oral sensation of a subject.

### [Background Art]

Learning Disorder (Specific Learning Disorder) is a condition in which an individual has difficulty learning a task in a specific area, even though his or her general intelligence is within the normal range, his or her vision (visual acuity) and hearing (auditory acuity) are not impaired, and there are no problems with the learning environment or motivation of the individual. There are various types of Learning Disorder, including Dyslexia, Dysgraphia, and Dyscalculia. Moreover, others are poor at or have difficulty with visuospatial cognition (the ability to accurately perceive the form and location of objects, including their position, shape, direction, and size).

Some individuals with Learning Disorder assert "the letters are shaking", "the text is wavy", or "the surface of the paper is shiny". Such symptoms are called Irlen syndrome, Meares-Irlen syndrome, or visual stress. It is known that these symptoms (Sense of sight: Vision) can be improved by using colored films and lenses. In particular, the use of colored lenses and films is effective for Irlen syndrome (see Non-Patent Documents 1 and 2).

Thus, Irlen syndrome and other likely disorders are thought to be potentially related to visual perception (bias in visual cognitive function), particularly photosensitivity.

### [Citation List]

### [Non Patent Document]

[Non Patent Document 1]
   Sandra Irlen et al., "A controlled field study of the use of coloured overlays on reading achievement", Australian Journal of Learning Disabilities, Volume 9, 2004 - Issue 2, Pages 14-22
[Non Patent Document 2]
   Keiko Kumagai et al., "The Research of Visual Characteristics of the Clients with Irlen Syndrome", Japanese Journal of Learning Disabilities, 2021 Volume 30 Issue 2, Pages 126-137

### [Summary of Invention]

### [Technical Problem]

As mentioned above, individuals with biased visual cognitive function (photosensitivity) may have a "sense of sight" that differs from that of healthy individuals. However, since they are born with this "sense of sight", it is difficult for them to realize that they are not normal. Thus, there are many people with biased visual cognitive function, even among those who are called to be healthy people.

Nevertheless, visual cognitive biases cannot be dealt with in hospitals and are only assessed in a few limited research facilities. Moreover, symptoms of bias in visual cognitive function vary widely, and there are large individual differences. Therefore, only those with specialized knowledge and experience can assess the biases of visual cognitive function.

A person whose visual cognitive function is biased often has an unbalanced diet. The person is hypersensitive or dull to oral sensation (taste, odor, hearing, touch (mouthfeel), etc.), and dislikes a specific food and does not eat it or prefers only a limited food.

For example, the person feels that batter of fried food or croquettes is stuck in a mouth and cannot be eaten. The person feels that carbonated water is stuck in the mouth, becomes painful, and cannot be taken. When chewing a shiitake mushroom or an eggplant, the person feels unpleasant by a spongy feeling. The sound of chewing food is unpleasant and unbearable. The person cannot eat a food having a specific smell such as mayonnaise, etc.

Information of different senses such as vision and taste strongly affect each other. A phenomenon in which perception in a certain sense changes by a function of complementing multisensory information (cross-modal interaction) is known.

The unbalanced diet may be caused by bias in a visual cognitive function adversely affecting oral sensation such as taste. It is presumed that the bias in the visual cognitive function disturbs sensory integration of vision and taste etc. and multisensory perception (integrated cognition).

Therefore, there is a possibility that the bias in the visual cognitive function can be assessed on the basis of sensory traits information regarding integration of vision and oral sensation of a subject.

In view of such circumstances, an object of the present invention is to provide a sensory traits information collecting method capable of collecting sensory traits information regarding integration of vision and oral sensation of a subject.

### [Solution to Problem]

A sensory traits information collecting method according to a first aspect of the present invention includes: a first tasting step of including, in a mouth of a subject, predetermined food and drink exhibiting predetermined oral sensation in a white light environment in which white light is incident on a retina of the subject; a predetermined light environment creating step of creating a predetermined light environment in which predetermined light having at least one of a spectral distribution and luminance different from that of the white light is incident on the retina of the subject; a second tasting step of again including the predetermined food and drink in the mouth of the subject in the predetermined light environment; an information reception step of receiving sensory traits information regarding oral sensation of the predetermined food and drink in the first tasting step and the second tasting step from the subject; and a collecting step of collecting the sensory traits information received from the subject.

The sensory traits information collecting method according to a second aspect is that of the first aspect including an aggregation step of aggregating the information obtained in the collecting step.

The sensory traits information collecting method according to a second aspect is that of the first aspect including an analysis table creation step of creating an analysis table in which the information obtained in the aggregation step is graphed or charted.

The sensory traits information collecting method according to a second aspect may also include, in the first aspect, an analysis step of analyzing the sensory traits related to oral sensation of the subject or a diagnosing step of diagnosing the sensory traits on the basis of the information obtained in the aggregation step.

The sensory traits information collecting method according to a second aspect is that of the first aspect in which the predetermined oral sensation is sensation with respect to a flavor including some of basic tastes.

The sensory traits information collecting method according to a second aspect is that of the first aspect in which the predetermined oral sensation is sensation with respect to a flavor including an aroma.

The sensory traits information collecting method according to a second aspect is that of the first aspect in which the predetermined oral sensation is sensation with respect to a texture including a mouthfeel.

The sensory traits information collecting method according to a second aspect is that of the first aspect in which the predetermined food and drink is a taste aqueous solution, a tablet, a granule, or a powder of sucrose, sodium chloride, tartaric acid, caffeine, or sodium glutamate.

The sensory traits information collecting method according to a second aspect is that of the first aspect in which the predetermined food and drink is lemonade confectionery exhibiting sweetness and acidity.

The sensory traits information collecting method according to a second aspect is that of the first aspect in which the predetermined food and drink is high-cacao chocolate exhibiting sweetness and bitterness.

The sensory traits information collecting method according to a second aspect is that of the first aspect in which the predetermined food and drink is carbonated water.

The sensory traits information collecting method according to a second aspect is that of the first aspect in which the predetermined light is yellow light having a dominant wavelength of 570 nm to 590 nm.

The sensory traits information collecting method according to a second aspect is that of the first aspect in which the predetermined light is magenta light having a complementary dominant wavelength of 500 nm to 570 nm.

The sensory traits information collecting method according to a second aspect is that of the first aspect in which the predetermined light is cyan light having a dominant wavelength of 470 nm to 530 nm.

The sensory traits information collecting method according to a second aspect is that of the first aspect in which the predetermined light is green light having a dominant wavelength of 500 nm to 570 nm.

The sensory traits information collecting method according to a second aspect is that of the first aspect in which the predetermined light has luminance of 0.001 to 5 cd/m².

The sensory traits information collecting method according to a second aspect is that of the first aspect in which at least one of spectral distribution and luminance of the predetermined light is changed, and then the steps from the predetermined light environment creating step to the collecting step are executed again.

The sensory traits information collecting method according to a second aspect is that of the first aspect in which, in the predetermined light environment creating step, a wearable optical device is attached to the subject.

The sensory traits information collecting method according to a second aspect is that of the first aspect in which the wearable optical device is glasses, contact lenses, or goggles.

The sensory traits information collecting method according to a second aspect is that of the first aspect in which the wearable optical device includes a yellow, magenta, cyan, green, or gray color lens.

The sensory traits information collecting method according to a second aspect is that of the first aspect in which the wearable optical device includes a color lens having a luminous transmittance of 90 to 50%.

### [Advantageous Effects of Invention]

The sensory traits information collecting method of the present invention can collect sensory traits information regarding integration of vision and oral sensation of a subject. Moreover, the sensory traits information regarding the integration of the vision and the oral sensation of the subject can be aggregated and analyzed.

### [Brief Description of Drawings]

FIG. 1 is a longitudinal sectional view illustrating a structure of a human eye, and illustrates (a) a white light environment and (b) a predetermined light environment.
FIG. 2(a) is a schematic diagram illustrating human photoreceptor cells, and FIG. 2(b) illustrates spectral sensitivity curves of the human photoreceptor cells.
FIG. 3 is an xy chromaticity diagram of a color space (CIE 1931).
FIG. 4 is a flowchart illustrating a sensory traits information collecting method according to an embodiment.
FIG. 5 is a diagram illustrating an answer form Q.
FIG. 6 is a diagram illustrating an SD chart analysis table D.

### [Description of Embodiments]

A sensory traits information collecting method according to an embodiment of the present invention will be explained with reference to the drawings.

The sensory traits information collecting method collects sensory traits information regarding integration of vision and oral sensation of a person with a biased visual cognitive function (subject).

In the sensory traits information collecting method, first, predetermined food and drink having a predetermined oral sensation (taste, flavor, and mouthfeel) is included in a mouth of the subject (oral sensation is given) under a white light environment.

Next, a predetermined light environment is created in which a "sense of sight" is easily changed for the person with the biased visual cognitive function, and the predetermined food and drink is included again in the mouth of the subject under the predetermined light environment.

Then, information regarding a mode/degree of change in oral sensation with respect to the predetermined food and drink under the white light environment and the predetermined light environment is collected from the subject. That is, sensory traits information regarding integration of vision and oral sensation of the subject is collected.

### [Photoreceptor Cell]

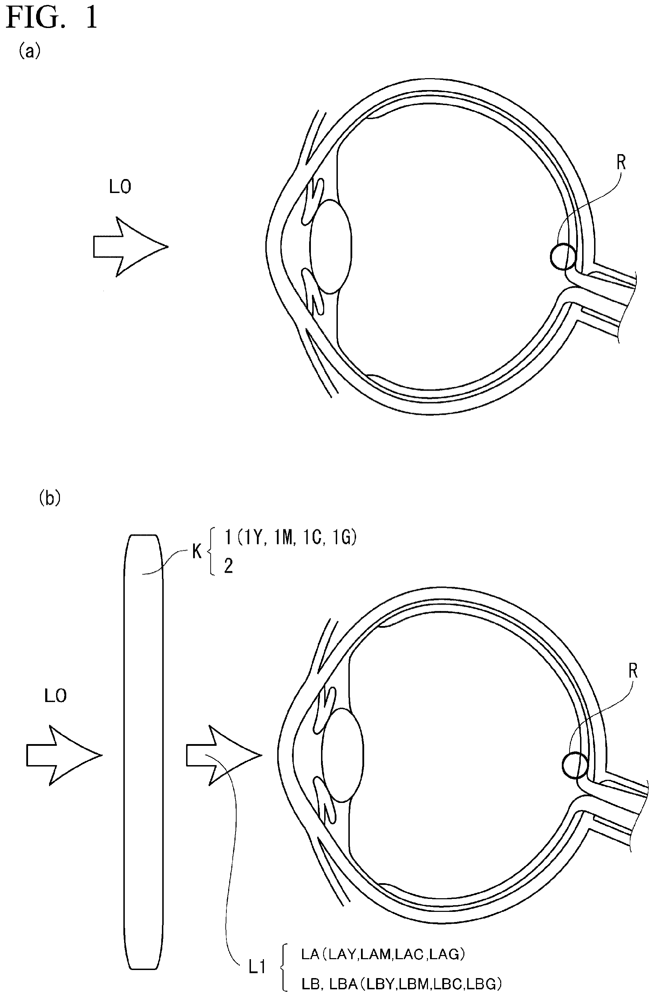

FIG. 1 is a longitudinal sectional view illustrating a structure of a human eye, and illustrates (a) a white light environment and (b) a predetermined light environment.

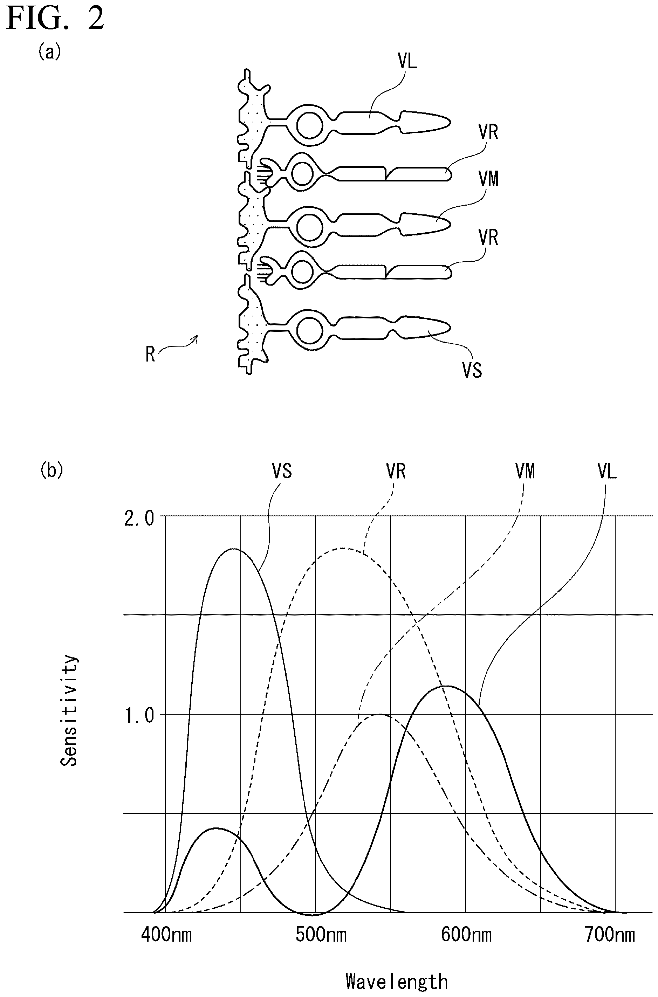

FIG. 2(a) is a schematic diagram illustrating human photoreceptor cells, and FIG. 2(b) illustrates spectral sensitivity curves of the human photoreceptor cells.

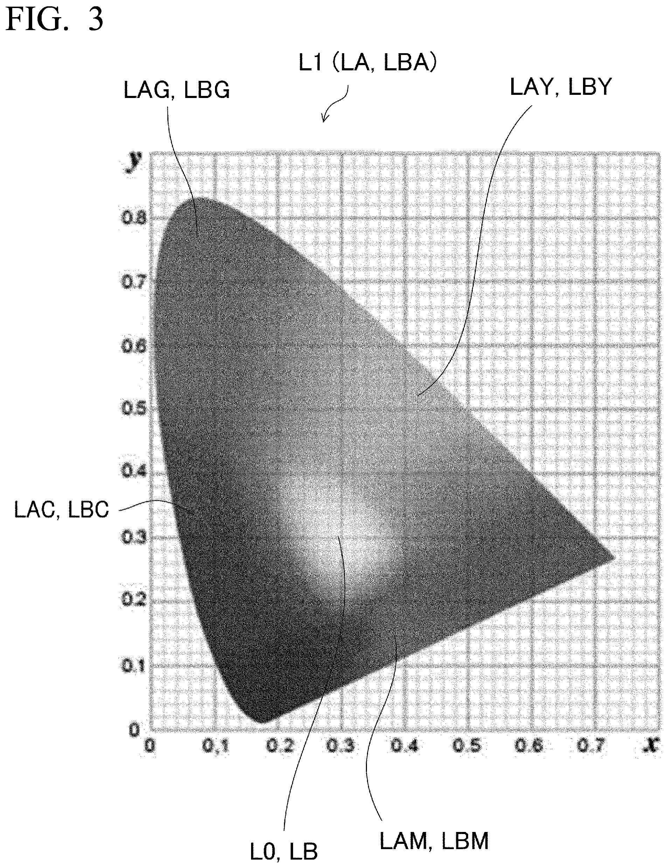

FIG. 3 is an xy chromaticity diagram of a color space defined by International Commission on Illumination (CIE 1931).

The photoreceptor cells in the human retina R include cone cells and rod cells. The cone cells are cone-shaped photoreceptor cells that are located near the central fovea of the retina R and detect color. The cone cells function in a bright place. The rod cells are rod-shaped photoreceptor cells that are located in the periphery of the central fovea and detect light. The rod cells function primarily in a dark place.

The visual function in a situation where the cone cells work (i.e., a situation in which there is a sufficient amount of light intensity) is called photopic vision. The photopic vision occurs under a light amount of the luminance 5 to 1000000 cd/m² (illuminance of 10 to 100000 lx).

The visual function in a situation where the rod cells work (i.e., a situation in which a light intensity is low) is called scotopic vision. The scotopic vision occurs under a light amount of the luminance 0.01 to 0.000001 cd/m² (illuminance of 0.001 to 0.01 lx).

The visual function in a situation where both the cone cells and the rod cells work (i.e., a situation in which the light amount is small but not completely darkness) is called mesopic vision. The mesopic vision is the combination of the photopic vision and the scotopic vision. The mesopic vision occurs under a light amount of the luminance 0.001 to 5 cd/m² (illuminance of 0.01 to 10 lx). The mesopic vision is defined as the visual function with luminance 0.005 to 5 cd/m² by the International Commission on Illumination (CIE) or as the visual function with luminance 0.001 to 3 cd/m² by the Illuminating Engineering Society (IES).

The cone cells are classified into three types corresponding to the three primary colors of light. Specifically, the cone cells include: long cone cells that react to light in a long wavelength range (around yellow); middle cone cells that react to light in a middle wavelength range (around yellow green); and short cone cells that react to light in a short wavelength range (around blue).

The long cone cells are also referred to as red cone cells. The middle cone cells are also referred to as green cone cells. The short cone cells are also referred to as blue cone cells.

A specific color is perceived according to a combination of intensities of stimulus received by each of the three types of cone cells (a relative ratio of excitation of the three types of cone cells).

Hereinafter, the long cone cells, the middle cone cells, the short cone cells, and the rod cells are also referred to as L photoreceptor cells VL, M photoreceptor cells VM, S photoreceptor cells VS, and R photoreceptor cells VR, respectively.

### [Visible Light]

The visible light is light having a wavelength of 380 to 780nm. A relationship (spectroscopic spectrum) between the wavelength and the color of visible light is substantially as follows.

Wavelength 380 to 430 nm: blue-violet, 430 to 460 nm: blue, 460 to 500 nm: blue-green, 500 to 570 nm: green, 570 to 590 nm: yellow, 590 to 610 nm: orange, 610 to 780 nm: red.

The sensitivity of a photoreceptor cell to visible light has wavelength dependency. Specifically, an absorption maximum wavelength of the L photoreceptor cell VL is around 558 nm, the absorption maximum wavelength of the M photoreceptor cell VM is around 531 nm, the absorption maximum wavelength of the S photoreceptor cell VS is around 419 nm, and the absorption maximum wavelength of the R photoreceptor cell VR is around 500 nm.

A peak wavelength at which the light-emission intensity of the visible light becomes maximum is different from the wavelength which is actually perceived by the eye. The wavelength of the color perceived by the eye is called a main wavelength or a dominant wavelength.

The light perceived as blue-violet has the dominant wavelength of around 400 nm (380 nm to 430 nm).

The light perceived as blue has the dominant wavelength of around 450 nm (430 nm to 470 nm).

The light perceived as cyan has the dominant wavelength of around 490 nm (470 nm to 530 nm).

The light perceived as green has the dominant wavelength of around 550 nm (530 nm to 570 nm).

The light perceived as yellow has the dominant wavelength of around 580 nm (570 nm to 590 nm).

The light perceived as red has the dominant wavelength of around 610 nm (590 nm to 780 nm).

The light perceived as magenta has a complementary dominant wavelength of around 550 nm (530 nm to 570 nm).

### (White Light L0)

The white light L0 is light in which light (colors) of all the wavelengths of the visible light are mixed almost equally, and which does not give any sense of color. The white light L0 may also be defined as a color of average daylight.

The white light L0 is light with luminance of 5 to 1000000 cd/m² and realizes the photopic vision by being incident on the retina R of the subject. The white light L0 is light in which all of the three types of cone cells (S photoreceptor cells VS, M photoreceptor cells VM, and L photoreceptor cells VL) present in the retina R react (are excited).

The white light L0 includes tint light (illumination light) called bulb color, warm white, day white, and daylight color, but all are light at positions along a blackbody locus in an xy chromaticity diagram of a color space. The white light L0 has brightness represented by color temperature [K].

An environment in which the white light L0 is incident on the retina R of the subject is referred to as a white light environment.

### (Predetermined Light L1)

The predetermined light L1 is visible light which is different from the white light L0 in at least one of spectral distribution and luminance. The predetermined light L1 includes light (colored light LA) having a spectral distribution different from that of the white light L0, light (gray light LB) with luminance different from that of the white light L0, and light (gray colored light LBA) having different spectral distribution and luminance from those of the white light L0.

Different spectral distribution means that the predetermined light L1 (the colored light LA and the gray colored light LBA) is colored light. Different luminance means that the predetermined light L1 (the gray light LB and the gray colored light LBA) is light with luminance 0.001 to 5 cd/m².

An environment in which the predetermined light L1 (LA, LB, and LBA) is incident on the retina R of the subject is referred to as a predetermined light environment.

### <Colored Light LA>

The light LA is light (colored light) with the luminance 5 to 1000000 cd/m², with light (color) of a part of the wavelength of the visible light.

The colored light LA is light at a position along a spectrum locus or a pure purple locus in the xy chromaticity diagram of the color space. The colored light has a color represented by a main wavelength (dominant wavelength) or a complementary main wavelength (complementary dominant wavelength).

The light LA is light which one or two of the three types of cone cells mainly react to. The light LA is any one of: yellow light LAY with the dominant wavelength of 570 nm to 590nm; magenta light LAM with the complementary dominant wavelength of 500 nm to 570 nm; cyan light LAC with the dominant wavelength of 470 nm to 530 nm; and green light LAG with the dominant wavelength of 500 nm to 570 nm.

The yellow light LAY is visible light which excites the L photoreceptor cell VL and the M photoreceptor cell VM and suppresses (sedates) the S photoreceptor cell VS.

The yellow light LAY includes, in addition to light having a peak wavelength of near 580 nm, light having a bottom wavelength of near 450 nm (complementary color of blue), and light having peak wavelengths of near 550 nm and near 610 nm (color mixture of green and red).

The yellow light LAY, when incident on the retina R, excites the L photoreceptor cell VL and the M photoreceptor cell VM and suppresses the S photoreceptor cell VS.

The magenta light LAM is visible light which excites the L photoreceptor cell VL and the S photoreceptor cell VS and suppresses the M photoreceptor cell VM.

The magenta light LAM includes, in addition to light having a bottom wavelength of near 550 nm (complementary color of green), light having peak wavelengths of near 450 nm and near 610 nm (color mixture of blue and red).

The cyan light LAC is visible light which excites the M photoreceptor cell VM and the S photoreceptor cell VS and suppresses the L photoreceptor cell VL.

The cyan light LAC includes, in addition to light having a peak wavelength of near 490 nm, light having a bottom wavelength of near 610 nm (complementary color of red) and light having peak wavelengths of near 450 nm and near 550 nm (color mixture of blue and green).

The green light LAG is visible light which excites the M photoreceptor cell VM and suppresses the L photoreceptor cell VL and the S photoreceptor cell VS.

The green light LAG includes, in addition to light having a peak wavelength of near 550 nm, light having bottom wavelengths of near 450 nm and near 610 nm (color mixture of yellow and cyan).

### <Gray Light LB>

The light LB is light (gray light) with luminance of 0.001 to 5 cd/m² in which light (colors) of all the wavelengths of the visible light is mixed almost equally. The gray light LB is light obtained by lowering the luminance of the white light L0 and is light in which color tones are difficult to perceive.

The gray light LB realizes the mesopic vision by entering the retina R of the subject. That is, the gray light LB is light to which the rod cells present in the retina R begin to react.

The rod cells are highly sensitive to light and darkness, which correspond to the intensity of light. The rod cells are not involved in color sensing. When the light with luminance of 5 cd/m² or less enters the retina R, it excites the rod cells and makes them sense the light and darkness.

The gray light LB excites both the cone cells (the L photoreceptor cells VL, the M photoreceptor cells VM, and the S photoreceptor cells VS) and the rod cells (the R photoreceptor cells VR) (mesopic vision).

The gray light LB has no color represented by a dominant wavelength or a complementary dominant wavelength. The dominant wavelength or the complementary dominant wavelength is a measure for only the colored light LA and is not applied to the gray light LB. That is, the gray light LB is light at a position along the blackbody locus in the xy chromaticity diagram of the color space, and has brightness represented by color temperature [K].

### <Gray Colored Light LBA>

The light LBA is light (gray colored light) with luminance of 0.001 to 5 cd/m² which includes light (color) of a part of the wavelength of the visible light. The light LBA includes gray-yellow light LBY, gray-magenta light LBM, gray-cyan light LBC, and gray-green light LBG.

The gray-yellow light LBY is light obtained by lowering luminance of the yellow light LAY. The gray-magenta light LBM is light obtained by lowering luminance of the magenta light LAM. The gray-cyan light LBC is light obtained by lowering luminance of the cyan light LAC. The gray-green light LBG is light obtained by lowering luminance of the green light LAG.

The gray colored light LBA is light obtained by combining the colored light LA and the gray light LB, has brightness represented by color temperature [K], and also has a color represented by a dominant wavelength or a complementary dominant wavelength.

### [Wearable Optical Device K]

The wearable optical device K is an optical device that causes the predetermined light L1 (light LA, LB, LBA) to enter the retina R of the subject. The wearable optical device K includes a first optical device 1 and a second optical device 2, and these are used alone or in combination.

The first optical device 1 and the second optical device 2 function as light sources for creating a predetermined light environment (predetermined light environment creating step S3).

### (First Optical Device 1)

The first optical device 1 is an optical device that causes the light LA (colored light) in which the cone cells react to enter the retina R. The first optical device 1 causes the light LA having luminance of 5 to 1000000 cd/m² to be incident on the retina R to achieve photopic vision. The first optical device 1 causes the light LA in which two of the three types of cone cells (S photoreceptor cells VS, M photoreceptor cells VM, and L photoreceptor cells VL) react to be incident on the retina R.

The light LA is any one of: yellow light LAY with the dominant wavelength of 570 nm to 590nm; magenta light LAM with the complementary dominant wavelength of 500 nm to 570 nm; cyan light LAC with the dominant wavelength of 470 nm to 530 nm; and green light LAG with the dominant wavelength of 500 nm to 570 nm.

The first optical device 1 includes an optical device 1Y, an optical device 1M, an optical device 1C, and an optical device 1G.

The optical device 1Y causes the yellow light LAY which excites the L photoreceptor cell VL and the M photoreceptor cell VM and suppresses the S photoreceptor cell VS to be incident on the retina R. That is, the optical device 1Y causes the yellow light LAY perceived as yellow (having a dominant wavelength of about 580 nm) to enter the retina R.

The optical device 1M causes the magenta light LAM which excites the L photoreceptor cell VL and the S photoreceptor cell VS and suppresses the M photoreceptor cell VM to be incident on the retina R. That is, the optical device 1M causes the magenta light LAM perceived as magenta (having a complementary dominant wavelength of about 550 nm) to be incident on the retina R.

The optical device 1C causes the cyan light LAC which excites the M photoreceptor cell VM and the S photoreceptor cell VS and suppresses the L photoreceptor cell VL to be incident on the retina R. That is, the optical device 1C causes the cyan light LAC perceived as cyan (having a dominant wavelength of about 490 nm) to enter the retina R.

The optical device 1G causes the green light LAG which excites the M photoreceptor cell VM and suppresses the L photoreceptor cell VL and the S photoreceptor cell VS to be incident on the retina R. That is, the optical device 1G causes the green light LAG perceived as green (having a dominant wavelength of about 550 nm) to enter the retina R.

### (Second Optical Device 2)

The second optical device 2 is an optical device that causes the gray light LB in which the rod cells (R photoreceptor cells VR) and all of the three types of cone cells react to be incident on the retina R.

The second optical device 2 causes the gray light LB to be incident on the retina R to realize mesopic vision. The gray light LB is light that does not give a tint of luminance 0.001 to 5 cd/m².

The first optical device 1 (optical device 1Y, optical device 1M, optical device 1C, and optical device 1G) and the second optical device 2 allow light to enter the retina R from most of a range (direction) of a field of view. The first optical device 1 and the second optical device 2 allow light to enter the retina R from the range of at least 50% or more of the field of view, preferably 80% or more of the field of view.

The first optical device 1 and the second optical device 2 are, for example, glasses (color lenses). The first optical device 1 and the second optical device 2 may be contact lenses, goggles, or the like.

For example, a yellow lens can be used as the optical device 1Y, a pink (magenta) lens can be used as the optical device 1Y, a sky blue (cyan) lens can be used as the optical device 1C, a green lens can be used as the optical device 1G, and a gray lens can be used as the second optical device 2.

The white light L0 is transmitted through the color lens to become the predetermined light L1 (light LA, LB, LBA), and the predetermined light is incident on the retina R of the subject.

Color density (reciprocal of luminous transmittance) of each color lens is preferably 5% to 60% (luminous transmittance of 95% to 40%) in order to suppress stimulation to the subject. In particular, the color density is preferably 10% to 50% (luminous transmittance of 90% to 50%). When the subject has a poor response, a color lens having a density of 60% to 85% (luminous transmittance of 40 to 15%) may be used.
[See JIS T 7331, JIS T 7333, ISO 14889, ISO 8980-3]

When the subject wears the wearable optical device such as glasses, contact lenses, or goggles, the predetermined light L1 (light LA, LB, LBA) enters the retina R from the range (direction) of at least 50% or more of the field of view of the subject.

The first optical device 1 and the second optical device 2 can be combined. The gray colored light LBA in which the rod cells (R photoreceptor cells VR) and two of the three types of cone cells (S photoreceptor cells VS, M photoreceptor cells VM, and L photoreceptor cells VL) react is incident on the retina R.

By overlapping the optical device 1Y and the second optical device 2, the gray-yellow light LBY perceived as yellow in the mesopic vision can be made incident on the retina R.

By overlapping the optical device 1M and the second optical device 2, it is possible to cause the gray-magenta light LBM perceived as magenta in the mesopic vision to be incident on the retina R.

By overlapping the optical device 1C and the second optical device 2, the gray-cyan light LBC perceived as cyan in the mesopic vision can be made incident on the retina R.

By overlapping the optical device 1G and the second optical device 2, the gray-green light LBG perceived as green in the mesopic vision can be made incident on the retina R.

### (Indoor Lighting Device C)

An indoor lighting device C is an optical device that causes the white light L0 to enter the retina R of the subject. The indoor lighting device C is installed in an indoor space where white light such as sunlight is substantially blocked, and an entire area of a room H is irradiated with the white light L0 by turning on the indoor lighting device C.

The indoor lighting device C is an illumination tool such as a lamp (light bulb, fluorescent lamp, LED, OLED), and functions as a light source that creates a white light environment (white light environment creating step S1). The indoor lighting device C emits the white light L0 with luminance of 5 to 1000000 cd/m². The white light L0 is incident on the retina R (a central visual field region and a peripheral visual field region) of the subject.

The indoor lighting device C may function as the first optical device 1 or the second optical device 2. That is, the predetermined light L1 (light LA, LB, LBA) may be emitted from the indoor lighting device C to be incident on the retina R of the subject.

### [Predetermined Food and Drink F]

Predetermined food and drink F is food and drink that can give the subject a predetermined oral sensation when it is included (put) in the mouth. That is, the predetermined food and drink F is food and drink that exhibits a taste, an odor (aroma), and a mouthfeel when included in the mouth.

The predetermined food and drink F is food and drink exhibiting some of basic tastes (basic five tastes) of a saltiness, acidity, sweetness, umami, and bitterness. In particular, the predetermined food and drink F is food and drink exhibiting (including) one or two of the basic tastes.

The predetermined food and drink F may be food and drink exhibiting an odor and a mouthfeel in addition to the basic taste, or food and drink exhibiting only an odor and a mouthfeel.

### (Taste Aqueous Solution: One Example of Commercially Available Taste Testing Kit)

For the predetermined food and drink F, a commercially available taste testing kit (taste aqueous solution) can be used.

Sweetness solute: sucrose; concentration 0.4% (weight per volume percent)
Saltiness solute: sodium chloride; concentration 0.13%
Acidity solute: tartaric acid; concentration 0.005%
Bitterness solute: caffeine; concentration 0.02%
Umami solute: sodium glutamate; concentration 0.05%

Granulated sugar may be used instead of sucrose. Citric acid may be used instead of tartaric acid.

It is also preferable to prepare each of the taste aqueous solutions having a concentration of about 2 to 10 times so that the subject can reliably recognize the basic taste.

The predetermined food and drink F is not limited to the taste aqueous solution, and may be a solid, a fluid, a gel (gummy), or the like. For example, tablets, granules, or powders containing a taste component may be used.

To exhibit a taste means to contain a component at a concentration exceeding a taste threshold. The taste threshold is the minimum concentration at which a taste can be recognized. The taste thresholds of the basic tastes are said to be 0.1 to 0.4% sweetness, 0.25% saltiness, 0.0012% acidity, 0.006% bitterness, and 0.03% umami.

**In** particular, the predetermined food and drink F is preferably food and drink exhibiting sweetness and acidity, or sweetness and bitterness at the same time. Examples thereof include an aqueous solution in which sucrose and tartaric acid are dissolved and an aqueous solution in which sucrose and caffeine are dissolved.

For example, lemonade confectionery containing glucose, corn starch (starch), and citric acid exhibits sweetness and acidity, and thus is suitable as the predetermined food and drink F. Further, chocolate (in particular, high cacao: cacao mass of 50% or more) exhibits sweetness and bitterness, and thus is suitable as the predetermined food and drink F.

### [Oral Sensation]

Oral sensation includes taste, odor (flavor), touch (mouthfeel), and hearing. The taste, odor, and hearing are special sensations obtained from a tongue, a nose, and ears (sensory organs). The flavor means an aroma (odor) or a taste that is felt when food or drink is put into the mouth.

The taste and odor have speed (reaction time), intensity, and persistence of sensation. Chemical stimuli such as a taste, an aroma (odor), and a flavor are also called flavors.

There are intensity and frequency of chewing sound, swallowing sound, and the like in the hearing.

The mouthfeel is sensation felt when food and drink is consumed, and is a cutaneous sensation (somatic sensation) in an oral cavity including teeth and a tongue. The mouthfeel includes temperature, mouthfeel, tongue touch, firmness/brittleness, viscosity, water absorbency, chewiness, adhesiveness/recoverability, a degree of breakage, change in moisture/oil content, how to spread into the oral cavity, feeling in a throat, swallowing easiness, and remaining feeling in the oral cavity or the throat, etc. of food and drink. These mouthfeels are also called textures.

It is said that a person's food preference (cognition) is formed by receiving flavors (taste, flavor), textures, temperature, a sound, and appearance of food and drink and comprehensively perceiving this sensory information.

The subject is caused to taste the predetermined food and drink F to impart oral sensation to the predetermined food and drink F.

The taste means to taste the predetermined food and drink F in the mouth, and is a behavior in which the subject feels not only taste but also mouthfeel, an aroma, a chewing sound, and the like.

The oral sensation to the predetermined food and drink F is quantitatively evaluated by a test similar to a sensory test. [Sensory tests: see JIS z 9080, JIS z 8144, ISO 8586, ISO 11132, etc.]

Evaluation of the oral sensation is not necessarily an analytical sensory evaluation that accurately evaluates a taste of the subject, but may be a preference sensory test that evaluates a taste preference of the subject. In addition, it is not always necessary to eat or swallow the predetermined food and drink F. The predetermined food and drink F may be taken in the mouth and then discharged.

In the evaluation of the oral sensation, it is required that prejudice or the like of the subject does not affect an evaluation result. Therefore, for example, an evaluation method such as a rating method or a semantic differential (SD) method is used.

### [Sensory Traits Information Collecting Method]

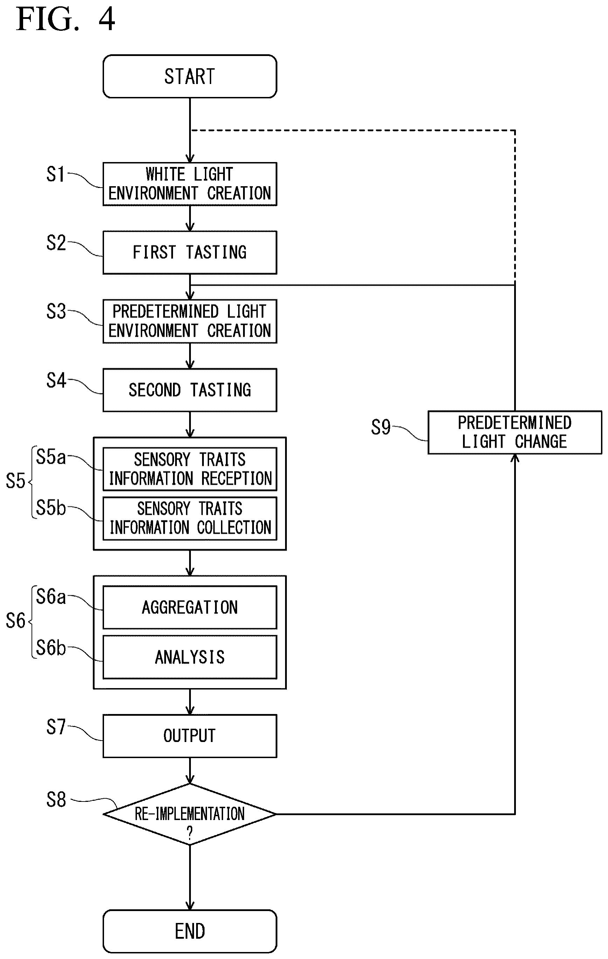

FIG. 4 is a flowchart illustrating a sensory traits information collecting method according to the embodiment.

The sensory traits information collecting method includes: a white light environment creating step S1; a first tasting step S2; a predetermined light environment creating step S3; a second tasting step S4; a sensory traits information reception and collecting step S5; and an aggregation/analysis step S6.

The method also includes: a re-implementation determination step S8; and a predetermined light changing step S9.

The sensory traits information reception and collecting step S5 and the aggregation/analysis step S6 are preferably performed using an IT device (not illustrated) such as a personal computer, a tablet terminal, or a smartphone.

For example, the personal computer includes an arithmetic processing unit (CPU), a storage unit (ROM, RAM, HDD, SSD, etc.), a display, a keyboard, a mouse, and the like. A printer or the like is connected to the personal computer. When the personal computer or the like is used, an outputting step S7 of outputting a result of aggregation or analysis from the printer or the like may be included.

### (White Light Environment Creating Step S1)

The sensory traits information collecting method is started in the white light environment in which the white light L0 is incident on the retina R of the subject. In the room H capable of blocking natural light (sunlight), the white light environment is created by turning on the indoor lighting device C arranged on a ceiling.

The white light environment may be an environment in which the subject does not feel glare. Luminance (illuminance) of light is preferably, for example, luminance of 200 to 3000 cd/m² (illuminance of 100 to 1000 lx) suitable for reading, working or the like.

The outdoor natural light (sunlight) is preferably avoided as the white light L0 because it has high luminance and the stimulus is too strong for the subject in many cases.

To allow the subject to acclimate to the white light environment (light adaptation), the subject is made to stay in the room H where the indoor lighting device C is on for about 1 minute or more (elapse of light adaptation time).

The white light L0 is absorbed to, reflected (diffusely reflected) on, or transmitted through a wall, a floor, or the like of the room H, and the reflected light or transmitted light is incident on the retina R of the subject. The white light L0 may be directly incident on the retina R of the subject from the indoor lighting device C.

### (First Tasting Step S2)

In the first tasting step S2, the subject is caused to have predetermined food and drink F having a predetermined oral sensation in the mouth under the white light environment. The subject is caused to taste the predetermined food and drink F in the mouth (tasting). Not only the taste but also the mouthfeel, odor, and sound (chewing sound and the like) of the predetermined food and drink F are felt. That is, the subject has a predetermined oral sensation.

As the predetermined food and drink F, for example, the following seven types (predetermined food and drink F1 to F7) are used.

The predetermined food and drink F1 is a sucrose containing aqueous solution (concentration of 0.4% or more), and exhibits sweetness.

The predetermined food and drink F2 is a sodium chloride-containing aqueous solution (concentration of 0.13% or more), and exhibits saltiness.

The predetermined food and drink F3 is a sucrose and tartaric acid-containing aqueous solution (sucrose: concentration of 0.4% or more, tartaric acid: concentration of 0.005% or more), and exhibits sweetness and acidity.

The predetermined food and drink F4 is a sucrose and caffeine-containing aqueous solution (sucrose: concentration of 0.4% or more, caffeine: concentration of 0.02% or more), and exhibits sweetness and bitterness.

The predetermined food and drink F5 is lemonade confectionery, and exhibits sweetness and acidity.

The predetermined food and drink F6 is high-cacao chocolate, and exhibits sweetness and bitterness.

The predetermined food and drink F5 and F6 also exhibit an odor (an aroma) and a mouthfeel in addition to the taste.

The predetermined food and drink F7 is carbonated water, and exhibits only a fizzy mouthfeel. Acidity sensitive cells in the oral cavity are activated by stimulation of carbonic acid, and acidity may be felt.

Not limited to the case of using all of the predetermined food and drink F1 to F7, some of them may be used, or other food and drink (particularly, food and drink that the subject does not like) may be used.

The predetermined food and drink F1 to F7 are not limited to the taste aqueous solutions, and may be tablets, granules, powders, or the like of a taste component.

### (Predetermined Light Environment Creating Step S3)

In the predetermined light environment creating step S3, the predetermined light environment in which the predetermined light L1 is incident on the retina of the subject is created.

While the indoor lighting device C is turned on, the subject is caused to wear the color lens glasses (first optical device 1 and second optical device 2). As a result, the white light L0 is transmitted through the first optical device 1 and the second optical device 2, becomes the predetermined light L1, and enters the retina R of the subject.

As the predetermined light L1, the light LA, which is colored light, is first selected. That is, it is any one of the yellow light LAY, the magenta light LAM, the cyan light LAC, and the green light LAG.

The yellow light LAY is the most preferable for the predetermined light L1. The subject is caused to wear the yellow lens glasses (optical device 1Y).

When the predetermined light L1 is incident on the retina R of the subject, two or one of the three types of cone cells (L photoreceptor cells VL, M photoreceptor cells VM, and S photoreceptor cells VS) mainly react.

The predetermined light L1 is directly incident on the retina R of the subject from the first optical device 1 or the second optical device 2.

In the white light environment creating step S1 (white light environment) and the predetermined light environment creating step S3 (predetermined light environment), the luminance (illuminance) of the light incident on the retina R is substantially the same.

In order to adapt the subject to the predetermined light environment (light adaptation), the subject is made to wait for about 1 minute or more (elapse of light adaptation time) after the subject wears the color lens glasses.

### (Second Tasting Step S4)

Next, the subject is caused to again have predetermined food and drink having the predetermined oral sensation in the mouth under the predetermined light environment.

After the first tasting step S2, the subject is allowed to drink fresh water or the like so that the taste of the predetermined food and drink F does not remain at the start of the second tasting step S4.

The predetermined food and drink F (F1 to F7) used in the first tasting step S2 is used as it is without changing the concentration, amount, temperature, and the like.

### (Sensory traits Information Reception and Collecting Step S5)

In the sensory traits information reception and collecting step S5, the subject is asked to answer whether or not a change (difference) has occurred in oral sensation of the predetermined food and drink F in the white light environment (first tasting step S2) and in the predetermined light environment (second tasting step S4) (the answer is received), and this information is collected. The subject is asked to answer a degree/mode of change in oral sensation. That is, sensory traits information regarding integration of vision and oral sensation is collected from the subject.

The sensory traits information reception and collecting step S5 may be executed in either the white light environment or the predetermined light environment. That is, the subject may be in a state of wearing the color lens glasses (first optical device 1 and second optical device 2) or in a state of removing the color lens glasses. The subject may answer while reconfirming the oral sensation of the predetermined food and drink F by wearing or removing the color lens glasses.

When the predetermined light L1 (yellow light LAY or the like) is incident on the retina R, a person having a biased visual cognitive function (photosensitivity) may feel that oral sensation (taste, mouthfeel, etc.) to the predetermined food and drink F is different from that in the white light environment. That is, integration (sensory integration or multisensory perception) of vision and oral sensation of the subject may change.

Specifically, it may be felt that a taste of a specific food and drink (predetermined food and drink F) has changed. The change in taste is not only a change in strength (depth) of taste but also a change in speed or persistence of taste. In food and drink exhibiting two tastes, the taste felt first is changed or the taste felt strongly is changed.

In addition, it may be felt that a mouthfeel of the specific food and drink (predetermined food and drink F) has changed. For example, a rough mouthfeel is changed to a crispy mouthfeel. For example, a sticky mouthfeel is changed to a crunchy mouthfeel. As described above, an unpleasant mouthfeel may be changed to a pleasant mouthfeel.

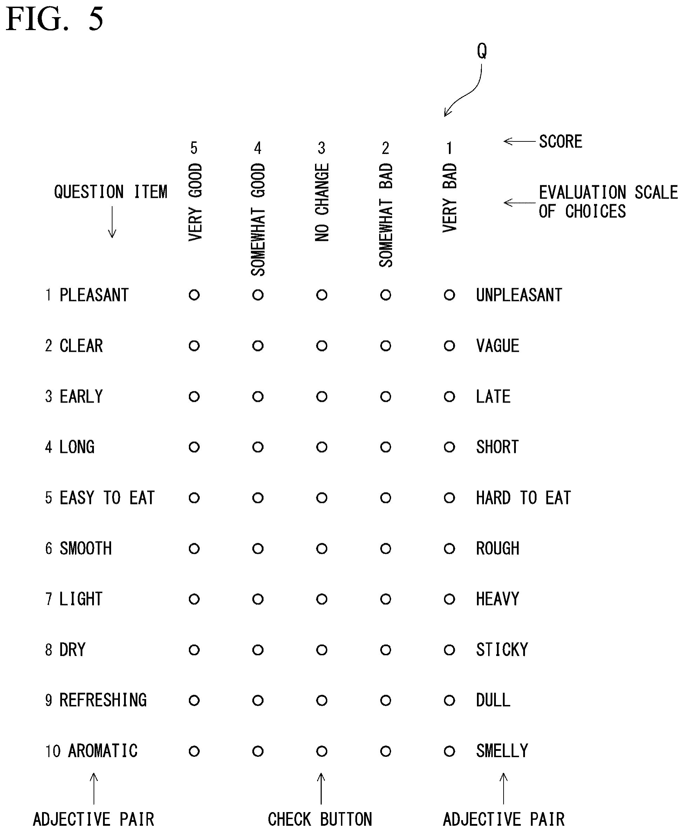

FIG. 5 is a diagram illustrating an answer form Q.

First, the answer form Q regarding a change in oral sensation of the predetermined food and drink F or the like is presented to the subject. When a personal computer is used, the answer form Q is displayed on a display of the personal computer (sensory traits information reception step S5a).

As the answer form Q, multiple choices are used (multiple-choice question method). The answers from the subjects are quantified by using the Likert Scale (Likert scale) or the like in which each evaluation scale stage of the multiple choices is used as a score.

For example, in the case of five choices, "very good" is set to 5 points, "somewhat good" is set to 4 points, "no change" is set to 3 points, "somewhat bad" is set to 2 points, and "very bad" is set to 1 point (rating method).

The evaluation scale of the multiple choices is set on the basis of the semantic differential method (SD method) or the like within the psycho-statistical method (psychological measurement method).

The following question items are set for the predetermined food and drink F1 to F7 and displayed on the display of the personal computer. The contents of each question item (adjective pair, evaluation scale score, and the like) are stored in the storage unit of the personal computer in advance.

Regarding the taste of the predetermined food and drink F1 to F7, for example, the following question items and adjective pairs are set.
Question item 1: change in overall taste and mouthfeel, adjective pair: "pleasant" - "unpleasant"

The question item 1 is a question related to whether the taste, mouthfeel, and the like of the predetermined food and drink F1 to F7 have changed.

Regarding the predetermined food and drink F1 to F6, for example, the following question items and adjective pairs are set.
Question item 2: change in depth and strength of taste, adjective pair: "clear" - "vague"
Question item 3: change in speed at which taste is sensed, adjective pair: "early" - "late"
Question item 4: change in time when taste is felt, adjective pair: "long" - "short"

For the predetermined food and drink F5 to F7, for example, the following question items and adjective pairs are set regarding a mouthfeel and a smell (an aroma).
Question item 5: change in mouthfeel, adjective pair: "easy to eat" - "hard to eat"
Question item 6: change in mouthfeel/tongue touch, adjective pair: "smooth" - "rough"
Question item 7: change in tooth touch/chewiness, adjective pair: "light" - "heavy"
Question item 8: how to spread into the oral cavity/change in feeling in the throat, adjective pair: "dry" - "sticky"
Question item 9: change in remaining feeling, adjective pair: "refreshing" - "dull"
Question item 10: change in odor, adjective pair: "aromatic" - "smelly"

Subsequently, the subject or the like operates a keyboard or a mouse to answer the question about the change in the oral sensation of the predetermined food and drink F. Specifically, the subject or the like enters a numerical value with the keyboard or click a check button with the mouse for each question item to choose the most applicable one of the five choices (single answer method).

For example, the subject gives an answer (numerical value input) about a change in oral sensation (taste, mouthfeel, and smell) when eating lemonade confectionery (predetermined food and drink F5) in a predetermined light environment.

Specifically, for the "change in the overall oral sensation" of the question item 1, when the subject feels that there was no change, the subject inputs "3".

On the other hand, when the subject feels that there was a change in the "change in the overall oral sensation", the subject inputs as follows. That is, the subject inputs "5" when the oral sensation has become very good and "4" when the oral sensation has become somewhat good. Also, the subject inputs "2" when the oral sensation has become somewhat bad and "1" when the oral sensation has become very bad.

That is, with the numerical value "3" as a reference, a larger numerical value means that the oral sensation has become better, and a smaller number means that the oral sensation has become worse.

The subject or the like gives four answers (numerical values) at the minimum and ten answers at the maximum for each of the predetermined food and drink F.

Four answers are obtained for the predetermined food and drink F1 to F4, ten answers are obtained for the predetermined food and drink F5 and F6, and seven answers are obtained for the predetermined food and drink F7.

As a result, a plurality of answers can be obtained to one or more predetermined food and drink F (sensory traits information collecting step S5b).

The answers to each of the predetermined food and drink F1 to F7 (raw data of sensory traits information regarding integration of vision and oral sensation) are stored (collected) in the storage unit of the personal computer.

### (Aggregation/Analysis Step S6)

In the aggregation/analysis step S6, first, the answer information obtained in the sensory traits information reception and collecting step S5 is aggregated, and presence/absence and a degree of the change in the oral sensation of the subject (aggregated information of the sensory traits information regarding the integration of the vision and the oral sensation) are acquired (aggregation step S6a).

Specifically, the arithmetic processing unit of the personal computer executes arithmetic processing on the answers (numerical values) stored in the storage unit and calculates aggregated information such as, for example, an average value, a mode value, a maximum value, a minimum value, a variance value, or a deviation. Moreover, a frequency at which each numerical value is input (answered) is calculated. The frequency at which a numerical value other than "3" is input (answered), the frequency at which "1" or "2" is input, and the frequency at which "4" or "5" is input are calculated.

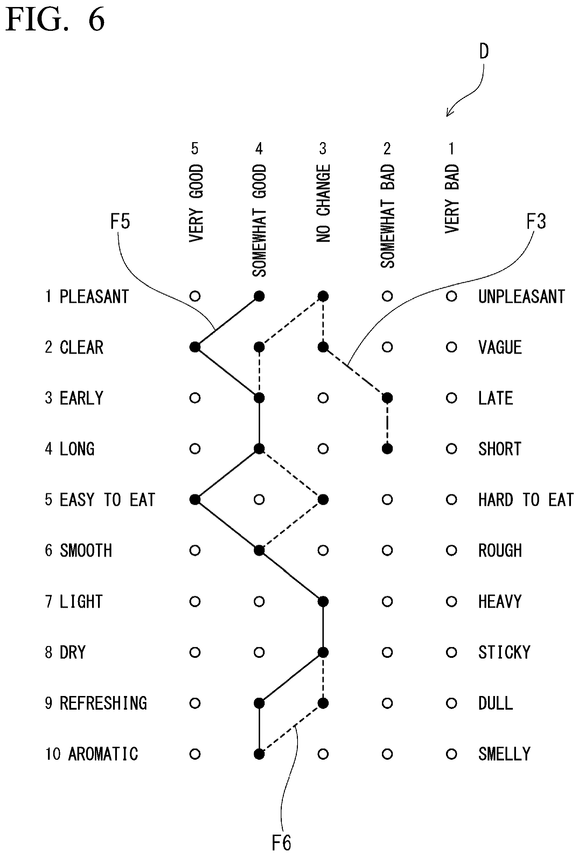

FIG. 6 is a diagram illustrating an SD chart analysis table D. This SD chart analysis table D is an example of chart-displaying the sensory traits information regarding the integration of the vision and the oral sensation of the predetermined food and drink F (F3, F5, F6) when the optical device 1Y (yellow light LAY) is used.

Subsequently, the arithmetic processing unit of the personal computer creates an analysis table (analysis information of the sensory traits information regarding the integration of the vision and the oral sensation) in which the question items and the answers (numerical values) are graphed and charted (analysis table creation step S6b).

Specifically, the arithmetic processing unit of the personal computer executes arithmetic processing on the aggregated information and creates analysis information (output image) such as the SD chart analysis table D. In addition to the SD chart analysis table D, the arithmetic processing unit may create an analysis table such as a pie chart, a laser chart, or a matrix.

Based on the information obtained in the aggregation step S6a or the analysis table created in the analysis table creation step S6b, an analysis step or a diagnosing step of analyzing or diagnosing the sensory traits related to the oral sensation of the subject may be performed.

### (Outputting Step S7)

Finally, in the outputting step S7, the aggregated information and the analysis information (results of various types of arithmetic processing) obtained in the aggregation/analysis step S6 are output to the outside. The arithmetic processing unit of the personal computer displays the aggregated information and the analysis information on the display of the personal computer as the degree of the change in the oral sensation of the subject (sensory traits information regarding the integration of the vision and the oral sensation). The aggregated information and the analysis information may be printed out by the printer.

Specifically, as the aggregated information, an average value, a mode value, a variance value or the like is output, for example. Moreover, an analysis table such as the SD chart is output as the analysis information.

### (Re-implementation Determination Step S8)

In the re-implementation determination step S8, it is determined whether the aforementioned sensory traits information collecting method will be re-executed or not.

For example, when the oral sensation of the subject with respect to the predetermined food and drink (F1 to F7) is hardly changed, the sensory traits information collecting method is executed again. In other words, it is determined whether to implement the sensory traits information collecting method would be executed again based on the aggregated information acquired in the aggregation/analysis step S6.

Specifically, the determination is made based on the frequency of the input of the numerical values other than "3" in the aggregated information. When the frequency is less than a threshold value (for example, 10%), the sensory traits information collecting method is executed again. On the other hand, when the frequency is equal to or more than the threshold value (for example, 10%), the sensory traits information collecting method is terminated. This threshold value can be arbitrarily set.

For example, it is determined whether the sensory traits information collecting method is executed again or not based on the number of types of gray colored light (light LA, LB, LBA) used in the predetermined light environment creating step S3.

The number of types of the optical devices 1 and 2 (the predetermined light L1) is stored in advance, and the sensory traits information collecting method is executed again until the number of times of the execution of the predetermined light environment creating step S3 matches the number of types of the optical devices 1 and 2.

Specifically, in a case of, for example, five types of the optical devices 1 and 2 (the predetermined light L1), if the predetermined light environment creating step S3 is executed less than five times, the sensory traits information collecting method is executed again. On the other hand, when the number of times of the executions of the predetermined light environment creating step S3 reaches five, the sensory traits information collecting method is terminated.

The number of types of the optical devices 1 and 2 (predetermined light L1) can be arbitrarily set.

### (Predetermined Light Changing Step S9)

When it is determined that the sensory traits information collecting method is to be re-executed, in the predetermined light changing step S9, the optical device (first optical device 1) is changed from the optical device 1Y (yellow light LAY) to the optical device 1M (magenta light LAM) or the like.

Then, in the predetermined light environment creating step S3, the magenta light LAM or the like is emitted to the subject from the optical device 1Y.

In the predetermined light environment creating step S3, the subject is allowed to wait for about 1 minute or more (elapse of light adaptation time) after the subject wears the optical device 1M so that the subject adapts to the changed predetermined light L1 (new predetermined light environment).

Subsequently, the second tasting step S4 to the outputting step S7 are executed.

In the re-execution of the sensory traits information collecting method, the white light environment creating step S1 and the first tasting step S2 may be omitted or may be re-executed.

In the predetermined light changing step S9, the optical device (first optical device 1) may be changed from the optical device 1Y (yellow light LAY) to the optical device 1C (cyan light LAC) or the optical device 1G (green light LAG). The order (priority order) of the four lights LA (the yellow light LAY, the magenta light LAM, the cyan light LAC, and the green light LAG) can be set arbitrarily.

According to the sensory traits and the like of the disorder of the subject, the second optical device 2 (the gray light LB or the gray colored light LBA) may be used for the optical device in addition to the first optical device 1 (the colored light LA) or instead of the first optical device 1.

When the light LB and LBA are used, only the indoor lighting device C is turned on at the luminance of 0.001 to 5 cd/m² in the room H in the predetermined light environment creating step S3.

When the light LB and LBA are used, in order to adapt the subject to the predetermined light environment (dark adaptation), the subject is made to wait for about 10 to 30 minutes in the room where the indoor lighting device C is on (elapse of dark adaptation time).

When the gray light (light LB) or gray colored light (light LBA) is incident on the retina R, a person having a biased visual cognitive function may feel that oral sensation (taste, mouthfeel, etc.) to the predetermined food and drink F is different from that in the white light environment. In addition, it is often felt that a mouthfeel of the specific food and drink (predetermined food and drink F) has changed. That is, integration (sensory integration or multisensory perception) of vision and oral sensation of the subject may change.

By following the aforementioned steps, the sensory traits information regarding the integration of the vision and the oral sensation of the subject can be collected without relying on a person having specialized knowledge or the like and with excluding subjectivity of the subject as much as possible. Moreover, the sensory traits information regarding the integration of the vision and the oral sensation of the subject can be aggregated and analyzed.

The oral sensation such as taste is strongly influenced by the photosensitivity of the subject. If there is a bias in photosensitivity (presence or absence, intensity, variation, or the like of sensitivity/torpor of sensation with respect to light or color), sensory integration of vision and taste etc., or multisensory perception (integrated cognition) is disturbed, and unbalanced diet is likely to occur.

Therefore, in the sensory traits information collecting method according to the embodiment of the present invention, the subject is caused to include the predetermined food and drink having the predetermined oral sensation in the mouth under the white light environment. Next, the predetermined light environment is created in which the "sense of sight" is easily changed for a person with a biased visual cognitive function, and the subject is caused to again include the predetermined food and drink in the mouth under the predetermined light environment. Then, the information regarding the mode/degree of the change in the oral sensation with respect to the predetermined food and drink under the white light environment and the predetermined light environment is collected from the subject.

As a result, the sensory traits information regarding the integration of vision and oral sensation of the subject is collected. At this time, the degree/mode of the change in the oral sensation are quantified and collected by using a statistical method such as the SD method. Therefore, the "sensory traits information regarding the integration of vision and oral sensation" of the subject can be collected objectively and quantitatively.

In addition, the "sensory traits information regarding the integration of the vision and the oral sensation" of the subject can be aggregated and analyzed. As a result, basic data for early diagnosis or the like of a biased visual cognitive function (photosensitivity) of the subject can be provided. Therefore, a training method for effectively improving and correcting the bias of the visual cognitive function of a person having a biased visual cognitive function (photosensitivity) can be created and executed.

Thus, the sensory traits information collecting method according to the embodiment of the present invention can be supplied inexpensively and easily in a wide range of fields including various industries, education, and traffic safety.

The present invention is not limited to the aforementioned embodiments but includes various modifications of the aforementioned embodiments without departing from the gist of the present invention. That is, the specific shapes, configurations and the like described in the embodiments are only examples and can be changed as appropriate.

In the sensory traits information collecting method of the present invention, the indoor lighting device C is not limited to the ceiling light. It may be a floor light, a desk light, a handy light or the like.

The white light L0 is not limited to artificial light emitted from various lighting fixtures but may be natural light (sunlight). For example, the sensory traits information collecting method of the present invention may be implemented in an environment in which the natural light enters the room through a window without using the indoor lighting device C or the like.

The first optical device 1 and the second optical device 2 are not limited to color lens glasses. The first optical device 1 and the second optical device 2 may be non-wearable optical devices.

For example, the white light environment and the predetermined light environment may be created, respectively, by changing the lighting color of the indoor lighting device C from white (white light L0) to yellow or the like (predetermined light L1). That is, the indoor lighting device C may also function as the first optical device 1 and the second optical device 2 (a light source of the predetermined light environment creating step S3).

The predetermined light L1 is not limited to artificial light emitted from various lighting fixtures, but natural light (sunlight) may be used. For example, a window pane in the room may be colored or a color filter may be attached to the window pane to create a predetermined light environment in the room H.

In addition to the yellow light LAY, the magenta light LAM, the cyan light LAC, and the green light LAG, red or blue light, for example, may be used as the predetermined light L1.

In addition to the gray light LB and the gray colored light LBA, light from which the light having a wavelength of 500 nm or less is cut or light from which the light having a wavelength of 400 nm or less is cut (anti-glare eyeglasses) may be used.

The predetermined light L1 is not limited to that being incident only on the peripheral visual field region in the retina R of the subject. The predetermined light L1 may be incident on the entire region (the central visual field region and the peripheral visual field region) of the retina R, or only on the central visual field region.

The sensory traits information collection is not limited to the desktop personal computer, but a laptop personal computer or a notebook personal computer may be used. A tablet terminal, a smartphone, or a mobile terminal may be used.

The sensory traits information reception and collecting step S5, the aggregation/analysis step S6, and the like may be performed without using the IT device. That is, the sensory traits information regarding the integration of vision and oral sensation may be directly heard from the subject, or the subject may directly write the sensory traits information on an answer sheet (answer form Q) of a paper medium.

The contents (multiple choices, question items, adjective pairs) of the above-described answer form Q are an example, and can be arbitrarily set according to the type, characteristics, and features of the predetermined food and drink F. The number of question items and the like for each predetermined food and drink F can also be arbitrarily set.

The sensory traits information reception and collecting step S5 is not limited to the case of being performed immediately after the second tasting step S4.

The sensory traits information reception and collecting step S5 may be performed immediately after the first tasting step S2 and immediately after the second tasting step S4. In this case, not the degree of change in taste, mouthfeel, or the like of the predetermined food and drink F but the taste, texture, or the like of the predetermined food and drink F in the first tasting step S2 and the second tasting step S4 are evaluated (scored) and answered (score method).

The outputting step S7 does not always have to be executed after each aggregation/analysis step S6. In the sensory traits information collecting method using a plurality of predetermined lights L1, the aggregation/analysis step S6 may be executed several times, and the outputting step S7 may be executed only once at the end.

### [Reference Signs List]

K Wearable optical device
1 First optical device
1Y Optical device (yellow lens glasses)
1M Optical device (magenta lens glasses)
1C Optical device (cyan lens glasses)
1G Optical device (green lens glasses)
2 Second optical device (gray lens glasses)
H Room
C Indoor lighting device
F Predetermined food and drink
F1 Sucrose containing aqueous solution (predetermined food and drink)
F2 Sodium chloride-containing aqueous solution (predetermined food and drink)
F3 Sucrose and tartaric acid-containing aqueous solution (predetermined food and drink)
F4 Sucrose and caffeine-containing aqueous solution (predetermined food and drink)
F5 Lemonade confectionery (predetermined food and drink)
F6 High-cacao chocolate (predetermined food and drink)
F7 Carbonated water (predetermined food and drink)
R Retina
VL L photoreceptor cell (Long cone cell)
VM M photoreceptor cell (Middle cone cell)
VS S photoreceptor cell (Short cone cell)
VR R photoreceptor cell (rod cell)
L0 White light
L1 Predetermined light
LA Colored light (predetermined light)
LAY Yellow light (predetermined light)
LAM Magenta light (predetermined light)
LAC Cyan light (predetermined light)
LAG Green light (predetermined light)
LB Gray light (predetermined light)
LBA Gray colored light (predetermined light)
LBY Gray-yellow light (predetermined light)
LBM Gray-magenta light (predetermined light)
LBC Gray-cyan light (predetermined light)
LBG Gray-green light (predetermined light)
Q Answer form
D SD chart analysis table

## Claims

1. A sensory traits information collecting method comprising:
a first tasting step of including, in a mouth of a subject, predetermined food and drink exhibiting predetermined oral sensation in a white light environment in which white light is incident on a retina of the subject;
a predetermined light environment creating step of creating a predetermined light environment in which predetermined light having at least one of a spectral distribution and luminance different from that of the white light is incident on the retina of the subject;
a second tasting step of again including the predetermined food and drink in the mouth of the subject in the predetermined light environment;
an information reception step of receiving sensory traits information regarding oral sensation of the predetermined food and drink in the first tasting step and the second tasting step from the subject; and
a collecting step of collecting the sensory traits information received from the subject.

2. The sensory traits information collecting method according to claim 1, comprising an aggregation step of aggregating the information obtained in the collecting step.

3. The sensory traits information collecting method according to claim 2, comprising an analysis table creation step of creating an analysis table in which the information obtained in the aggregation step is graphed or charted.

4. The sensory traits information collecting method according to claim 1, wherein the predetermined oral sensation is sensation with respect to a flavor including some of basic tastes.

5. The sensory traits information collecting method according to claim 1, wherein the predetermined oral sensation is sensation with respect to a flavor including an aroma.

6. The sensory traits information collecting method according to claim 1, wherein the predetermined oral sensation is sensation with respect to a texture including a mouthfeel.

7. The sensory traits information collecting method according to claim 1, wherein the predetermined food and drink is a taste aqueous solution, a tablet, a granule, or a powder of sucrose, sodium chloride, tartaric acid, caffeine, or sodium glutamate.

8. The sensory traits information collecting method according to claim 1, wherein the predetermined food and drink is lemonade confectionery exhibiting sweetness and acidity.

9. The sensory traits information collecting method according to claim 1, wherein the predetermined food and drink is high-cacao chocolate exhibiting sweetness and bitterness.

10. The sensory traits information collecting method according to claim 1, wherein the predetermined food and drink is carbonated water.

11. The sensory traits information collecting method according to claim 1, wherein the predetermined light is yellow light having a dominant wavelength of 570 nm to 590 nm.

12. The sensory traits information collecting method according to claim 1, wherein the predetermined light is magenta light having a complementary dominant wavelength of 500 nm to 570 nm.

13. The sensory traits information collecting method according to claim 1, wherein the predetermined light is cyan light having a dominant wavelength of 470 nm to 530 nm.

14. The sensory traits information collecting method according to claim 1, wherein the predetermined light is green light having a dominant wavelength of 500 nm to 570 nm.

15. The sensory traits information collecting method according to claim 1, wherein the predetermined light has luminance of 0.001 to 5 cd/m².

16. The sensory traits information collecting method according to claim 1, wherein at least one of spectral distribution and luminance of the predetermined light is changed, and then the steps from the predetermined light environment creating step to the collecting step are executed again.

17. The sensory traits information collecting method according to claim 1, wherein, in the predetermined light environment creating step, a wearable optical device is attached to the subject.

18. The sensory traits information collecting method according to claim 17, wherein the wearable optical device is glasses, contact lenses, or goggles.

19. The sensory traits information collecting method according to claim 17, wherein the wearable optical device includes a yellow, magenta, cyan, green, or gray color lens.

20. The sensory traits information collecting method according to claim 19, wherein the wearable optical device includes a color lens having a luminous transmittance of 90 to 50%.
